# EUROPÄISCHE PATENTSCHRIFT

(11) **EP 0 388 576 B1**
(45) Veröffentlichungstag und Bekanntmachung des Hinweises auf die Patenterteilung: **15.09.1993**
(21) Anmeldenummer: 89810858.4
(22) Anmeldetag: 09.11.1989
(51) Int. Cl.: A61F 2/30, A61C 8/00

(54) **Metallisches Implantat**
Metallic implant
Implant métallique

(30) Priorität: 23.03.1989 CH 1091/89
(43) Veröffentlichungstag der Anmeldung: 26.09.1990
(73) Patentinhaber: INSTITUT STRAUMANN AG, CH-4437 Waldenburg (CH)
(72) Erfinder: Steinemann, Samuel G., Prof. Dr., CH-1025 St. Sulpice (CH); Claes, Lutz, Prof., D-7900 Ulm (DE)
(74) Vertreter: Ullrich, Gerhard, Dr.

(56) Entgegenhaltungen:
- EP-A- 0 202 031
- DE-A- 2 717 615
- FR-A- 2 421 595
- GB-A- 2 045 083
- US-A- 3 605 123

## Beschreibung

Es sind schon die verschiedensten Implantate, die zum Einsetzen in einen Knochen dienen, bekannt geworden. Zu den Grösseren gehören die Hüftgelenkprothesen, zu den kleineren die in den Kiefer einzuschraubenden Stifte für den Aufbau von künstlichen Zähnen. Solche Implantate bestehen sehr oft aus Titan oder aus Zirkon oder aus Niob oder aus Tantal oder aus gewebeverträglichen Legierungen, deren Hauptbestandteil aus einem dieser Elemente gebildet wird. Bei all diesen Implantaten besteht das Problem darin, die nötigen Massnahmen zu treffen, dass die Knochensubstanz die Möglichkeit erhält, sich rasch und dauerhaft mit der Implantatoberfläche zu verbinden. Man spricht von Verankerung des Implantats, auch Osteointegration.

In der Zusammenfassung einer Studie des Arbeitskreises für Implantate des deutschen Verbandes für Materialprüfung, vorgetragen am 17. November 1987 in Berlin, wird folgendes festgestellt:
"Es wurde in kombinierten Zugfestigkeitsprüfungen und histomophometrischen Untersuchungen nachgewiesen, dass glatte Titan-Implantate im Knochen kein auf Zug belastbares Interface ausbilden. Es bedarf einer Oberflächenrauhigkeit von mehr als 20 µm, um durch mechanische Retentionen einen zugfesten Knochen-Implantat-Verbund aufzubauen. Regelmässige geometrische Oberflächenmuster und unregelmässige, poröse Strukturen können die Zugfestigkeit des Knochen-Titan-Interface besonders bei zusätzlichem Sandstrahlen verbessern."

Es ist heute üblich, diejenige Oberfläche eines Implantats, mit der der Knochen zusammenwachsen soll, mit einer Titan-Plasmaschicht zu überziehen oder auf ihr durch Sandstrahlen oder Schneiden eines feinen Gewindes eine Oberflächenrauhigkeit zu erzeugen. Die Kenntnisse über die Nachteile der so behandelten Oberflächen sind eben so verbreitet wie die Überzeugung, dass diese Art der Oberflächenrauhigkeit nötig ist, um eine gute Haftung zwischen Knochen und Implantat zu erhalten. Die Nachteile bestehen im wesentlichen darin, dass die mechanisch spröde Plasmaschicht eine Neigung zum Abplatzen oder Abspringen hat und dass die Oberflächen, die durch ein Strahlmittel aufgerauht sind, durch dieses Strahlmittel (meist Korund) verunreinigt sind. Versuche, die Oberfläche mit einem Beizmittel, wie HF + HN0₃ anschliessend zu reinigen und aufzuhellen, ergaben als Resultat ein wesentlich schlechteres Einwachsen und Verankern der Knochensubstanz auf der Oberfläche des Implantats.

Aus der GB-A-2 045 083 ist nun ein mit einer porösen Oberfläche versehenes, zum Einsetzen in einen Knochen bestimmtes Implantat bekannt, dessen Oberfläche eine Mikrorauhigkeit mit einer Porengrösse in der Grössenordnung von 0,01 µm bis 0,3 µm aufweist.

Es wurde nun gefunden, dass ein Implantat, dessen poröse Oberfläche eine Mikrorauhigkeit mit einer Porengrösse in der Grössenordnung von 2 µm und weniger aufweist, dann die besten Voraussetzungen aufweist um ein rasches und dauerhaftes Einwachsen zu gewährleisten, wenn diese Mikrorauhigkeit einer Makrorauhigkeit mit einer Porengrösse über 10 µm, vorzugsweise von über 20 µm, überlagert ist. Die vorliegende Erfindung betrifft nun derartige Implantate. Die erfindungsgemässen Implantate lassen sich dadurch herstellen, dass ein Implantat aus einem gewebeverträglichen Material, wie z.B. aus Titan oder aus einer Titanbasislegierung mit einem Strahlmittel, wie etwa Korund, zur Erzeugung einer Makrorauhigkeit gestrahlt wird und dass man nach dem Strahlen zur Erzeugung einer Mikrorauhigkeit auf den Körper eine reduzierenden Säure, wie z.B. HF, HCl oder HCl + H₂S0₄ einwirken lässt. Das ergibt eine Oberfläche, in welcher die Knochensubstanz hinreichend rasch so einwächst, dass sie sich mit ihr dauerhaft verbindet. Untersuchungen haben ergeben, dass das vor allem die Folge der Kombination von Makro- und Mikrostruktur ist. Dadurch, dass das Anätzen in reduzierender Säure nach dem Strahlen ausgeführt wird, werden die in der Oberfläche eingesprengten Partikel des Strahlmittels herausgelöst und die Oberfläche enthält keinerlei Fremdkörper. Ihre erfindungsgemässe Struktur ist keine Beschichtung sondern Bestandteil des Implantats.

Nachfolgend werden die Resultate von verschiedenen Versuchen dargelegt, die den Fortschritt zeigen, der sich mit der Erfindung erreichen lässt.

Als Implantat wurden jeweils 6 Titan-Schrauben 4,5 x 12 mm (Knochenschrauben) verwendet, deren Oberfläche wie folgt behandelt worden ist:
- I: Elektro-poliert.
- II: Standardbehandlung: fein gestrahlt, dann gebeizt in HF + HN0₃, anschliessend anodisiert.
- III: Plasmabeschichtet.
- IV: Gestrahlt mit Korund mittelfein (0,12 - 0,25 mm), gebeizt in HF + HN0₃.
- V: Gestrahlt mit Korund grob (0,25 - 0,50 mm), gebeizt in HF + HN0₃.
- VI: Gestrahlt mit Korund grob (0,25 - 0,50 mm), gebeizt in HCl + H₂S0₄.

Die Messungen der Rauhigkeit mit einem Standard-Testgerät ergeben folgende Resultate:

| | RZ | RA | RT |
|---|---|---|---|
| I | - | - | - |
| II | 8,4/6.5 | 0,97/1,01 | 10,3/9,0 |
| III | 53/30 | 5,9/5,1 | 76/41 |
| IV | 6,7/5,5 | 0,84/0,79 | 7,7/6,3 |
| V | 17/16 | 2,3/2,6 | 20/21 |
| VI | 23/19 | 3,0/3,1 | 28/22 |
| RZ = gemittelte Rauhtiefe RA = mittlerer Rauhwert RT = maximale Rauhtiefe | | | |

Hiezu ist zu bemerken, dass das Auflösungsvermögen des Testgerätes durch die Geometrie der Diamantspitze des Tasters begrenzt ist. Diese weist folgende Abmessungen auf:
Konuswinkel 85°
Radius der Verrundung an der Spitze: 5 µm.

Daher lässt sich mit einem solchen Gerät zwar die Rauhigkeit, nicht aber die Mikrorauhigkeit messen. Infolge dessen wurden zur Prüfung und Beurteilung der Oberfläche Aufnahmen mit dem Rasterelektronenmikroskop gemacht.

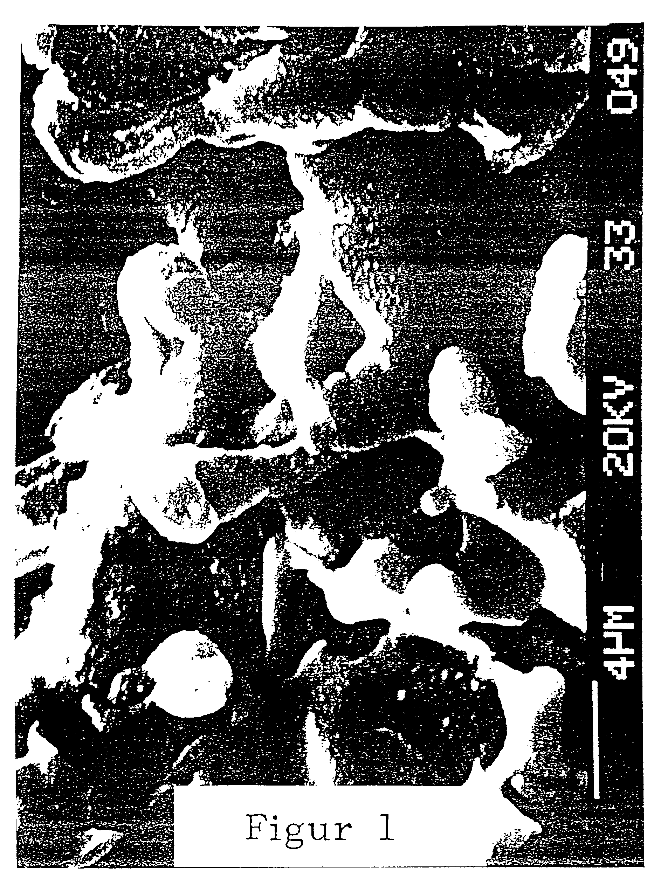

Die Figur 1 zeigt die Oberfläche der nach III behandelten Schraube,

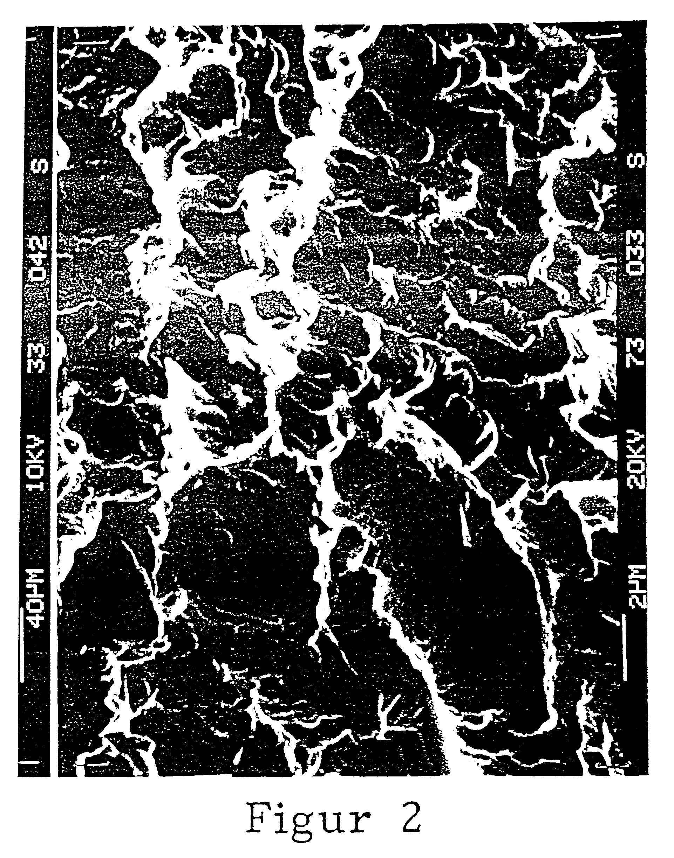

die Figur 2 zeigt die Oberfläche nach VI behandelten Schraube, beide im gleichen Massstab, und
die Figur 3 zeigt die Resultate der mit den 6 verschiedenen Schrauben durchgeführten Versuche.

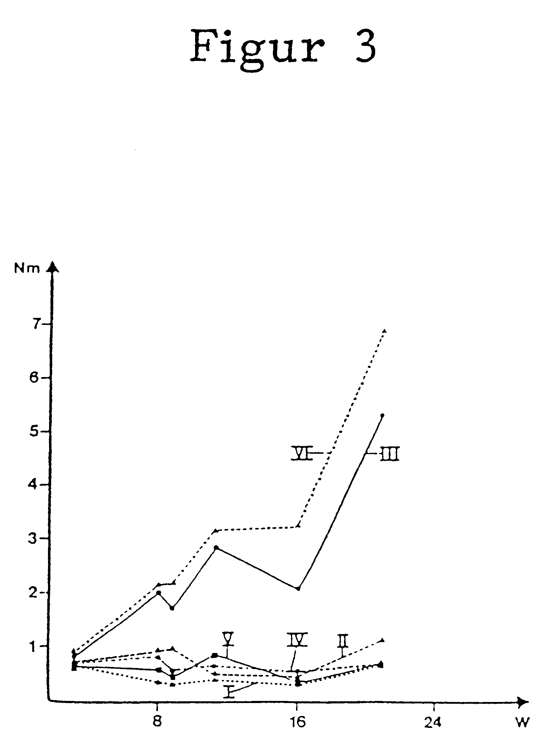

Die Schrauben wurden dem Versuchstier (Schaf) in die Tibia eingesetzt und zwar mit einem Drehmoment von 1 N.m. Nach einer Liegedauer von 3, 8, oder 9 Wochen und länger (z.B.) 11, 16 und 21 Wochen, wurde das Drehmoment gemessen, das zum Lösen der Schrauben vom Knochen nötig war. Aus der Figur 3 ergibt sich, dass die gemäss I, II, IV und V behandelten Schrauben nach 21 Wochen keine significant bessere Haftung am Knochen hatten, als nach 3 Wochen. Ganz im Unterschied dazu zeigten die Versuche, dass die an sich bekannte und bei Implantaten bereits verwendete Plasma-Beschichtung ein Einwachsen des Knochens in die Schicht während einer langen Zeit derart fördert, dass die Qualität der Verbindung mindestens bis zum Ende der 21-wöchigen Beobachtungszeit stufenweise zunimmt. Dieses Resultat der systematischen Versuche an einer Plasmaschicht stimmt mit dem Erfahrungsschatz der Praktiker überein.

Völlig neu, unerwartet und überraschend ist jedoch das Resultat, das sich mit der durch eine Ätzung erzeugten, die Makrorauhigkeit überlagernden Mikro-Rauhigkeit (Zustand VI) erreichen lässt: Die Verankerung beträgt bis das 7-fache des Eindrehmomentes und ist etwa 30% mehr als jene der Plasma-Schicht. Auch nimmt die Haftung sozusagen kontinuierlich, also praktisch unterbruchsfrei zu. Das ist unerwartet, denn dieses Resultat steht ganz im Gegensatz zur bisher in der Fachliteratur vertretenen Meinung, die dahin geht, dass eine Oberflächenrauhigkeit von mehr als 20 µm vorhanden sein müsse, damit der Knochen sich optimal mit dem Implantat verbinden könne. Das Vorhandensein der Mikrostruktur lässt sich auch ohne Elektronen-Mikroskop prüfen: Wenn die Oberfläche dunkel oder schwarz erscheint, ist die Mikrorauhigkeit kleiner als die Wellenlänge des sichtbaren Lichts oder etwa in der Grössenordnung von dieser Wellenlänge (etwa 0,5 µm). Die in reduzierender Säure geätzten Knochenschrauben haben schwarzes Aussehen. Für Implantate der Zahnchirurgie wird gewöhnlich Titan oder Tantal verwendet. Diese Metalle sind korrosionsresistent und verhalten sich im lebenden Gewebe träge, d.h. erzeugen keine Fremdkörperreaktionen. Es ist bekannt, dass Zirkon und Niob gleichermassen gewebeverträglich sind. Implantate der Orthopädie brauchen Metalle höherer mechanischer Festigkeit. Neue Werkstoffe zu diesem Zweck haben Zusammensetzungen wie sie z.B. in der CH-A-539 118 beschrieben sind. Bevorzugte Legierungen sind z.B. Ti7Nb6Al, Ti5Al2,5Fe, Ti15Mo5Zr3Al (Zahlen geben Gewichtsprozente der Legierungskomponenten). Für alle diese Metalle kann die Mikrorauhigkeit der Oberfläche in reduzierender Säure hergestellt werden.

## Patentansprüche

1. Zum Einsetzen in einen Knochen bestimmtes Implantat, dessen Oberfläche eine Mikrorauhigkeit mit einer Porengrösse in der Grössenordnung von 2 µm und weniger aufweist, dadurch gekennzeichnet, dass diese Mikrorauhigkeit einer Makrorauhigkeit mit Porengrössen der Oberfläche von mehr als 10 µm, vorzugsweise von mehr als 20 µm, überlagert ist.

2. Verfahren zur Herstellung eines Implantats nach Anspruch 1, dadurch gekennzeichnet, dass die Oberfläche des zur Bildung des Implantates dienenden metallischen Körper mit einem Strahlmittel, vorzugsweise Korund, zur Erzeugung einer Makrorauhigkeit gestraht wird und dass man nach dem Strahlen zur Erzeugung einer Mikrorauhigkeit auf den Körper eine reduzierende Säure einwirken lässt.

3. Verfahren nach Anspruch 2, dadurch gekennzeichnet, dass man als reduzierende Säure HCl oder HF oder eine Mischung von HCl und H₂SO₄ verwendet.

4. Verfahren nach Anspruch 2 oder Anspruch 3, dadurch gekennzeichnet, dass man die reduzierende Säure in kochendem Zustand verwendet.

## Claims

1. Implant intended for use in a bone, the surface of the implant having a micro-roughness with a pore size in the order of magnitude of 2µm and less, characterised thereby that this micro-roughness is superimposed on a macro-roughness with pore sizes of the surface of more than 10µm, preferably of more than 20µm.

2. Method for the manufacture of an implant according to claim 1, characterised thereby that the surface of the metallic body serving for the formation of the implant is blasted with a blasting medium, preferably corundum, for the production of a macro-roughness and that after the blasting a reducing acid is allowed to act on the body for the production of a micro-roughness.

3. Method according to claim 2, characterised thereby that HC1 or HF or a mixture of HC1 and H₂SO₄ is used as reducing acid.

4. Method according to claim 2 or claim 3, characterised thereby that the reducing acid is used in boiling state.

## Revendications

1. Implant destiné à l'implantation intra-osseuse et dont la surface présente une microrugosité ayant une grosseur de pores de l'ordre de 2 µm et moins, caractérisé en ce que cette microrugosité est superposée à une macrorugosité de la surface ayant une grosseur de pores de plus de 10 µm, de préférence de plus de 20 µm.

2. Procédé de fabrication d'un implant selon la revendication 1, caractérisé en ce que la surface du corps métallique servant à la formation de l'implant est traitée par projection d'un agent, de préférence du corindon, pour que soit créée une macrorugosité et qu'après cette projection, on laisse agir un acide réducteur sur le corps pour que soit créée une microrugosité.

3. Procédé selon la revendication 2, caractérisé en ce qu'on utilise comme acide réducteur HCl ou HF ou un mélange de HCl et H₂SO₄.

4. Procédé selon la revendication 2 ou la revendication 3, caractérisé en ce qu'on utilise l'acide réducteur à l'état d'ébullition.
